Europäisches Patentamt

European Patent Office

Office européen des brevets

(19)

(11) **EP 1 041 984 B1**

(12) **FASCICULE DE BREVET EUROPEEN**

(45) Date de publication et mention
de la délivrance du brevet:
**22.10.2003 Bulletin 2003/43**

(21) Numéro de dépôt: **98963579.2**

(22) Date de dépôt: **14.12.1998**

(51) Int Cl.$^7$: **A61K 31/4418**, A61K 47/48,
A61K 47/40

(86) Numéro de dépôt international:
**PCT/FR98/02712**

(87) Numéro de publication internationale:
**WO 99/033466 (08.07.1999 Gazette 1999/27)**

(54) **COMPOSITION PHARMACEUTIQUE BUVABLE**

TRINKBARES ARZNEIMITTEL

PHARMACEUTICAL COMPOSITION FOR ORAL ADMINISTRATION

(84) Etats contractants désignés:
**AT BE CH CY DE DK ES FI FR GB GR IE IT LI LU
MC NL PT SE**
Etats d'extension désignés:
**AL LT LV MK RO SI**

(30) Priorité: **24.12.1997 FR 9716529**

(43) Date de publication de la demande:
**11.10.2000 Bulletin 2000/41**

(73) Titulaire: **SANOFI-SYNTHELABO
75013 Paris (FR)**

(72) Inventeurs:
• **ALEMAN, Claude
F-34080 Montpellier (FR)**
• **BASTARD, Philippe
F-34080 Montpellier (FR)**
• **BONNEL, Marielle
F-34660 Couronsec (FR)**
• **BREUL, Thierry
F-34080 Montpellier (FR)**

(74) Mandataire: **Tsitini-Souleau, Maria et al
Sanofi-Synthelabo,
Département Brevets,
174, Avenue de France
75013 Paris (FR)**

(56) Documents cités:
**US-A- 5 378 709**

**Description**

La présente invention a pour objet une composition pharmaceutique orale buvable contenant le chlorhydrate de 1-[2-(2-naphtyl)éthyl]-4-(3-trifluorométhylphényl)1,2,3,6-tétrahydropyridine.

Plus particulièrement l'invention se rapporte à une composition buvable du chlorhydrate de 1-[2-(2-naphtyl) éthyl]-4-(3-trifluorométhylphényl)1,2,3,6-tétrahydropyridine en solution aqueuse avec de la β-cyclodextrine à pH acide.

La 1-[2-(2-naphtyl)éthyl]-4-(3-trifluorométhylphényl)1,2,3,6-tétrahydropyridine, ci-après désignée par son numéro de code SR 57746, et ses sels pharmaceutiquement acceptables, notamment son chlorhydrate, ont été décrits dans EP 0 101 381 comme étant des agents anorexigènes et, par la suite, comme anti-anxiodépresseurs (US 5,026,716), anticonstipants (US 5,109,005), neurotrophiques (US 5,270,320), anti-radicaux libres (US 5,292,745), cardioprotecteurs (US 5,378,709) et comme agents utiles dans le traitement de la sclérose latérale amyotrophique (WO 97/15304).

Dans certains de ces documents, il est indiqué que le SR 57746 peut être administré sous des formes pharmaceutiques convenables, y compris sous forme de complexe avec des cyclodextrines. Aucun complexe de ce type n'a toutefois jamais été décrit.

La faible solubilité du SR 57746 et de ses sels dans l'eau, notamment le chlorhydrate, (0,03 mg/ml) ainsi que l'instabilité des solutions aqueuses ainsi formées, représentent un problème sérieux pour l'administration et le stockage de solutions contenant ce composé. Ce problème se manifeste de façon encore plus importante lorsqu'on décide de préparer une solution aqueuse buvable pouvant être avalée aisément par des patients présentant des problèmes de déglutition.

Des études préliminaires menées avec les solubilisants classiques, y compris des cyclodextrines (ci-après désignées CD), ont conduit en général soit à une solubilisation insuffisante, soit à un dégradation partielle du SR 57746. Par exemple, des résultats décevants ont été obtenus avec la 2-hydroxypropyl-β-CD, l'α-CD et la γ-CD. Les dérivés méthylés (comme par exemple la RAMEB-CD, de l'anglais "randomized methylated β-CD") semblaient donner des résultats intéressants mais leur utilisation dans des compositions pharmaceutiques n'est pas pour l'instant autorisée par les pharmacopées européenne et américaine.

Il a été maintenant trouvé qu'on peut solubiliser des grandes quantités de chlorhydrate de SR 57746, en obtenant des solutions aqueuses stables, en utilisant la β-cyclodextrine, cette solubilisation étant améliorée aux pH acides.

Plus particulièrement on a vérifié que les composants ci-dessus, en quantités relatives données, permettent d'obtenir des solutions aqueuses stables dans le temps même en conditions de températures extrêmes.

Ainsi la présente invention a pour objet une solution aqueuse à base de chlorhydrate de 1-[2-(2-naphtyl)éthyl]-4-(3-trifluorométhylphényl)-1,2,3,6-tétrahydropyridine (chlorhydrate de SR 57746) caractérisée en ce qu'elle comprend

- une quantité non nulle de β-cyclodextrine (β-CD) inférieure ou égale à 50 mg/ml ;
- une quantité non nulle de chlorhydrate de SR 57746 (en mg/ml) inférieure ou égale au dixième de la quantité de β-CD exprimée en mg/ml ;
- un acide ou un tampon pharmaceutiquement acceptable pour assurer un pH inférieur ou égal à 3 ;

étant entendu que pour une quantité de β-CD allant de 30 à 50 mg/ml, la quantité de chlorhydrate de SR 57746 vérifie l'équation :

$$\text{quantité de chlorhydrate de SR57746 (mg/ml)} \geq [\frac{\text{quantité de } \beta\text{ - CD (mg / ml)}}{10}\text{-3}]$$

Le chlorhydrate de SR57746 peut être préparé selon les méthodes décrites dans EP 101381 ou WO 98/28273.

La β-CD à utiliser selon l'invention est une β-CD conforme aux tests des Pharmacopées européenne et américaine.

Les acides pharmaceutiquement acceptables utilisables selon la présente invention sont par exemple les acides acétique, citrique, tartrique, ascorbique, lactique, succinique, fumarique.

Ces acides peuvent être utilisés tels quels ou inclus dans des systèmes tampons.

Des exemples de tampons utilisables selon l'invention sont les systèmes acide acétique/acétate de sodium ou potassium; acide tartrique/tartrate de sodium ou potassium; acide lactique/lactate de sodium ou potassium; et acide ascorbique/ascorbate de sodium ou potassium.

Pour la préparation de la solution de l'invention, l'acide citrique, sous forme non-hydraté ou hydraté, notamment l'acide citrique monohydraté, est particulièrement avantageux.

Selon un aspect préféré, la présente invention concerne une solution aqueuse à base de chlorhydrate de 1-[2-(2-naphtyl)éthyl]-4-(3-trifluorométhylphényl)-1,2,3,6-tétrahydropyridine (chlorhydrate de SR 57746) caractérisée en ce qu'elle comprend

- une quantité non nulle de β-CD inférieure ou égale à 50 mg/ml
- une quantité non nulle de chlorhydrate de SR 57746 (en mg/ml) inférieure ou égale à un dixième de la quantité de β-CD exprimée en mg/ml
- une quantité d'un acide citrique allant de 0,1 mg/ml à 200 mg/ml étant entendu que pour une quantité de β-CD allant de 30 à 50 mg/ml, la quantité de chlorhydrate de SR 57746 vérifie l'équation :

$$\text{quantité de chlorhydrate de SR 57746 (mg/ml)} \geq [\frac{\text{quantité de } \beta \text{ - CD (mg / ml)}}{10} \text{-3}]$$

Des solutions préférées selon la présente invention comprennent une quantité de chlorhydrate de SR 57746 allant de 0,1 à 2 mg/ml.

Selon un aspect préféré, les solutions de l'invention comprennent une quantité de β-CD comprise entre 5 et 15 mg/ml.

Selon un aspect particulièrement préféré les solutions de l'invention comprennent une quantité de chlorhydrate de SR 57746 allant de 0,1 à 1,1 mg/ml, une quantité de β-CD comprise entre 5 et 15 et une quantité d'acide citrique allant de 1 à 100 mg/ml, de préférence de 5 à 50 mg/ml, avantageusement d'environ 10 mg/ml.

Parmi ces solutions, celles comprenant 0,55 mg/ml ou 1,1 mg/ml de chlorhydrate de SR57746, environ 10 mg/ml de β-CD et environ 10 mg/ml d'acide citrique sont des plus avantageuses.

Il a été trouvé, par des essais de RMN de couplage à 2 dimensions, que le SR 57746 dans l'eau, forme avec la β-CD un complexe comprenant 2 molécules de β-CD pour une molécule de SR 57746.

La stoechiométrie de ce complexe a été confirmée par des études de microcalorimétrie de titration en solution aqueuse en présence d'acide citrique monohydraté à environ 10 mg/ml.

Il a été également trouvé que deux molécules de β-CD encapsulent la molécule de SR 57746 en solution aqueuse aux deux extrémités opposées du composé SR 57746 et que le complexe ainsi obtenu est non seulement très soluble mais aussi très stable en solution aqueuse.

Le complexe formé entre une molécule de SR 57746 et deux molécules de β-CD est nouveau est constitue une objet ultérieur de la présente invention.

La solution peut être préparée, selon les techniques usuelles, en mélangeant dans de l'eau, suivant un ordre quelconque, le chlorhydrate de SR 57746, la β-CD et l'acide ou le système tampon choisi, dans les quantités prévues par l'invention, et en agitant le mélange jusqu'à dissolution complète des constituants.

La solution peut ensuite être purifiée, par exemple par ultrafiltration, éventuellement passée en autoclave, selon les techniques usuelles, puis stockée telle quelle ou distribuée dans des récipients mono ou pluridose.

La solution selon la présente invention peut être utilisée sous forme d'unités de dosage contenant une quantité efficace de principe actif.

Ainsi, selon un autre de ses aspects, la présente invention concerne une composition pharmaceutique buvable, en unités de dosage, caractérisée en ce qu'elle comprend une solution aqueuse telle que définie ci-dessus, dans laquelle le chlorhydrate de SR 57746 est présent dans une quantité de 0,5 à 10 mg par unité de dosage, de préférence de 1 à 5 mg par unité de dosage.

La solution et la composition de l'invention peuvent éventuellement comprendre des agents édulcorants ou aromatisants pour en corriger le goût.

Des compositions pharmaceutiques particulièrement avantageuses sont indiquées dans le tableau I

Tableau I

| Compositions pharmaceutiques en unités de dosage | | |
| --- | --- | --- |
| Chlorhydrate de SR 57746 | 2,2 mg | 4,4 mg |
| β-CD | 40 mg | 40 mg |
| Acide citrique monohydraté | 42 mg | 42 mg |
| Eau                                qsp | 4 ml | 4 ml |

Les compositions pharmaceutiques selon la présente invention, qu'elles soient ou non passées en autoclave, se sont révélées très stables au stockage, dans les conditions suivantes :

- une température comprise entre 5°C et 40°C ;
- un stockage de 3 mois.

Les compositions de l'invention, en unités de dosage, peuvent être conditionnées selon la pratique habituelle, par exemple en flacons de verre ou de polycarbonate, polychlorure de vinyle, polyéthylène ou polypropylène et fermées par des bouchons pharmaceutiquement acceptables par exemple en élastomère de chlorobutyle ou bromobutyle, éventuellement téflonnés et recouverts, le cas échéant, avec une capsule adaptée.

Exemple

Solution de SR 57746 à 0,5 mg/ml

On mélange à la température ambiante, à l'air libre ou à l'abri de l'oxygène ou sous une couverture d'azote, dans un ordre quelconque, 220 mg de chlorhydrate de SR 57746 (correspondant à 200 mg de SR 57746 base libre), 4 g de β-CD (Roquette Frères, conforme aux tests des Pharmacopées européenne et américaine), 4,2 g d'acide citrique monohydraté dans 400 ml d'eau et on agite jusqu'à dissolution complète.

On distribue 4 ml de solution dans des flacons de verre blanc de 9 ml; on ferme avec des bouchons en élastomère de chlorobutyle et recouvre le bouchon avec une capsule d'aluminium.


**Revendications**

1.   Solution aqueuse à base de chlorhydrate de 1-[2-(2-naphtyl)éthyl]-4-(3-trifluorométhylphényl)-1,2,3,6-tétrahydropyridine (chlorhydrate de SR 57746) **caractérisée en ce qu'**elle comprend:

  - une quantité non nulle de β-CD inférieure ou égale à 50 mg/ml;
  - une quantité de chlorhydrate de SR 57746 (en mg/ml) inférieure ou égale au dixième de la quantité de β-CD exprimée en mg/ml;
  - un acide ou un tampon pharmaceutiquement acceptable pour assurer un pH inférieure ou égale à 3

     étant entendu que pour une quantité de β-CD allant de 30 à 50 mg/ml, la quantité
     de chlorhydrate de SR 57746 vérifie l'équation:

$$\text{quantité de chlorhydrate de SR57746 (mg/ml)} \geq [\frac{\text{quantité de } \beta \text{ - CD (mg / ml)}}{10}\text{-3}]$$

2.   Solution selon la revendication 1 où l'acide pharmaceutiquement acceptable est choisi parmi les acides acétique, tartrique, ascorbique, lactique, succinique, fumarique et citrique tels quels, ou inclus dans des systèmes tampons.

3.   Solution selon la revendication 2 où l'acide pharmaceutiquement acceptable est l'acide citrique.

4.   Solution selon la revendication 3 où l'acide citrique est présent en quantités allant de 0,1 mg/ml à 200 mg/ml.

5.   Solution selon la revendication 1 **caractérisée en ce qu'**elle comprend une quantité de chlorhydrate de SR 57746 allant de 0,1 à 2 mg/ml.

6.   Solution selon la revendication 1 **caractérisée en ce qu'**elle comprend une quantité de β-CD comprise entre 5 et 15 mg/ml.

7.   Solution selon la revendication 1 **caractérisée en ce qu'**elle comprend une quantité de chlorhydrate de SR 57746 allant de 0,1 à 1,1 mg/ml, une quantité de β-CD comprise entre 5 et 15 mg/ml et une quantité d'acide citrique allant de 1 à 100 mg/ml.

8.   Solution selon la revendication 7 **caractérisée en ce qu'**elle comprend 0,55 mg/ml de chlorhydrate de SR 57746, une quantité de β-CD d'environ 10 mg/ml et une quantité d'acide citrique d'environ 10 mg/ml.

9.   Solution selon la revendication 7 **caractérisée en ce qu'**elle comprend 1,1 mg/ml de chlorhydrate de SR 57746, une quantité de β-CD d'environ 10 mg/ml et une quantité d'acide citrique d'environ 10 mg/ml.

10. Composition pharmaceutique buvable, en unités de dosage, **caractérisée en ce qu'**elle comprend la solution aqueuse selon la revendication 1, dans laquelle le chlorhydrate de SR 57746 est présent dans une quantité de 0,5 à 10 mg par unité de dosage.

11. Composition selon la revendication 10, dans laquelle le chlorhydrate de SR 57746 est présente dans une quantité de 1 à 5 mg par unité de dosage.

12. Composition selon la revendication 11, **caractérisée en ce qu'**elle comprend la solution aqueuse selon la revendication 2.

13. Composition selon la revendication 11, **caractérisée en ce qu'**elle comprend la solution aqueuse selon la revendication 3.

14. Composition selon la revendication 11, **caractérisée en ce qu'**elle comprend la solution aqueuse selon la revendication 4.

15. Composition selon la revendication 11, **caractérisée en ce qu'**elle comprend la solution aqueuse selon la revendication 5.

16. Composition selon la revendication 11, **caractérisée en ce qu'**elle comprend la solution aqueuse selon la revendication 6.

17. Composition selon la revendication 11, **caractérisée en ce qu'**elle comprend la solution aqueuse selon la revendication 7.

18. Composition pharmaceutique buvable, selon la revendication 11, **caractérisée en ce qu'**elle comprend 2,2 mg de chlorhydrate SR 57746, 40 mg de β-CD, 42 mg d'acide citrique monohydraté et de l'eau, le volume total de la composition étant de 4 ml.

19. Composition pharmaceutique buvable, selon la revendication 11, **caractérisée, en ce qu'**elle comprend 4,4 mg de chlorhydrate de SR 57746, 40 mg de β-CD, 42 mg d'acide citrique monohydraté et de l'eau, le volume total de la composition étant de 4 ml.

20. Complexe formé entre une molécule de SR57746 et deux molécules de β-CD.

**Patentansprüche**

1. Wässrige Lösung auf der Grundlage von 1-[2-(2-Naphthyl)-ethyl]-4-(3-trifluormethylphenyl)-1,2,3,6-tetrahydropyridin-Hydrochlorid (Hydrochlorid von SR 57746), **dadurch gekennzeichnet, daß** sie:

   - eine von null verschiedene Menge von β-CD von weniger oder gleich 50 mg/ml;
   - eine Menge des Hydrochlorids von SR 57746 (in mg/ml), die kleiner ist oder gleich einem Zehntel der Menge von β-CD ausgedrückt in mg/ml;
   - eine pharmazeutisch annehmbare Säure oder einen pharmazeutisch annehmbarer Puffer zur Sicherstellung eines pH-Wertes von weniger oder gleich 3 umfaßt,

   wobei es sich versteht, daß bei einer Menge von β-CD, die sich von 30 bis 50 mg/ml erstreckt, die Menge des Hydrochlorids von SR 57746 die folgende Gleichung erfüllt:

$$\text{Menge des Hydrochlorids von SR 57746 (mg/ml)} \geq \left[\frac{\text{Menge von β-CD (mg/ml)}}{10} - 3\right]$$

2. Lösung nach Anspruch 1, in der die pharmazeutisch annehmbare Säure ausgewählt ist aus Essigsäure, Weinsäure, Ascorbinsäure, Milchsäure, Bernsteinsäure, Fumarsäure und Citronensäure, die so oder in Form von Puffersystemen vorliegen.

3. Lösung nach Anspruch 2, worin die pharmazeutisch ahnenmbare Säure die Citronensäure ist.

4. Lösung nach Anspruch 3, worin die Citronensäure in einer Menge von 0,1 mg/ml bis 200 mg/ml vorliegt.

5. Lösung nach Anspruch 1, **dadurch gekennzeichnet, daß** sie eine Menge des Hydrochlorids von SR 57746 von 0,1 bis 2 mg/ml umfaßt.

6. Lösung nach Anspruch 1, **dadurch gekennzeichnet, daß** sie eine Menge von β-CD umfaßt, die zwischen 5 und 15 mg/ml liegt.

7. Lösung nach Anspruch 1, **dadurch gekennzeichnet, daß** sie eine Menge des Hydrochlorids von SR 57746 von 0,1 bis 1,1 mg/ml, eine Menge von β-CD zwischen 5 und 15 mg/ml und eine Menge der Citronensäure von 1 bis 100 mg/ml umfaßt.

8. Lösung nach Anspruch 7, **dadurch gekennzeichnet, daß** sie 0,55 mg/ml des Hydrochlorids von SR 57746, eine Menge von β-CD von etwa 10 mg/ml und eine Menge von Citronensäure von etwa 10 mg/ml umfaßt.

9. Lösung nach Anspruch 7, **dadurch gekennzeichnet, daß** sie 1,1 mg/ml des Hydrochlorids von SR 57746, eine Menge von β-CD von etwa 10 mg/ml und eine Menge der Citronensäure von etwa 10 mg/ml umfaßt.

10. Trinkbare pharmazeutische Zubereitung in Form von Dosiseinheiten, **dadurch gekennzeichnet, daß** sie eine wässrige Lösung nach Anspruch 1 umfaßt, in der das Hydrochlorid von SR 57746 in einer Menge von 0,5 bis 10 mg pro Dosiseinheit enthalten ist.

11. Zubereitung nach Anspruch 10, **dadurch gekennzeichnet, daß** das Hydrochlorid von SR 57746 in einer Menge von 1 bis 5 mg pro Dosiseinheit enthalten ist.

12. Zubereitung nach Anspruch 11, **dadurch gekennzeichnet, daß** sie die wässrige Lösung nach Anspruch 2 umfaßt.

13. Zubereitung nach Anspruch 11, **dadurch gekennzeichnet, daß** sie die wässrige Lösung nach Anspruch 3 umfaßt.

14. Zubereitung nach Anspruch 11, **dadurch gekennzeichnet, daß** sie die wässrige Lösung nach Anspruch 4 umfaßt.

15. Zubereitung nach Anspruch 11, **dadurch gekennzeichnet, daß** sie die wässrige Lösung nach Anspruch 5 umfaßt.

16. Zubereitung nach Anspruch 11, **dadurch gekennzeichnet, daß** sie die wässrige Lösung nach Anspruch 6 umfaßt.

17. Zubereitung nach Anspruch 11, **dadurch gekennzeichnet, daß** sie die wässrige Lösung nach Anspruch 7 umfaßt.

18. Trinkbare pharmazeutische Zubereitung nach Anspruch 11, **dadurch gekennzeichnet, daß** sie 2,2 mg des Hydrochlorids von SR 57746, 40 mg β-CD, 42 mg Citronensäure-Monohydrat und Wasser umfaßt, wobei das Gesamtvolumen der Zubereitung 4 ml beträgt.

19. Trinkbare pharmazeutische Zubereitung nach Anspruch 11, **dadurch gekennzeichnet, daß** sie 4,4 mg des Hydrochlorids von SR 57746, 40 mg β-CD, 42 mg Citronensäure-Monohydrat und Wasser umfaßt, wobei das Gesamtvolumen der Zubereitung 4 ml beträgt.

20. Komplex, gebildet aus einem Molekül SR 57746 und zwei Molekülen β-CD.

**Claims**

1. Aqueous solution based on 1-[2-(2-naphthyl)ethyl]-4-(3-trifluoromethylphenyl)-1,2,3,6-tetrahydropyridine hydrochloride (hydrochloride of SR 57746), **characterized in that** it comprises:

    - a non-zero amount of β-CD of less than or equal to 50 mg/ml;
    - an amount of SR 57746 hydrochloride (in mg/ml) of less than or equal to one tenth of the amount of β-CD expressed in mg/ml;
    - a pharmaceutically acceptable acid or buffer to give a pH of less than or equal to 3

    given that, for an amount of β-CD ranging from 30 to 50 mg/ml, the amount of SR 57746 hydrochloride fits the equation:

amount of SR 57746

$$\text{hydrochloride (mg/ml)} \geq [\frac{\text{amount of } \beta\text{-CD (mg/ml)}}{10} - 3]$$

2.  Solution according to Claim 1, in which the pharmaceutically acceptable acid is chosen from acetic acid, tartaric acid, ascorbic acid, lactic acid, succinic acid, fumaric acid and citric acid, as they are or included in buffer systems.

3.  Solution according to Claim 2, in which .the pharmaceutically acceptable acid is citric acid.

4.  Solution according to Claim 3, in which the citric acid is present in amounts ranging from 0.1 mg/ml to 200 mg/ml.

5.  Solution according to Claim 1, **characterized in that** it comprises an amount of SR 57746 hydrochloride ranging from 0.1 to 2 mg/ml.

6.  Solution according to Claim 1, **characterized in that** it comprises an amount of $\beta$-CD of between 5 and 15 mg/ml.

7.  Solution according to Claim 1, **characterized in that** it comprises an amount of SR 57746 hydrochloride ranging from 0.1 to 1.1 mg/ml, an amount of $\beta$-CD of between 5 and 15 mg/ml and an amount of citric acid ranging from 1 to 100 mg/ml.

8.  Solution according to Claim 7, **characterized in that** it comprises 0.55 mg/ml of SR 57746 hydrochloride, an amount of $\beta$-CD of about 10 mg/ml and an amount of citric acid of about 10 mg/ml.

9.  Solution according to Claim 7, **characterized in that** it comprises 1.1 mg/ml of SR 57746 hydrochloride, an amount of $\beta$-CD of about 10 mg/ml and an amount of citric acid of about 10 mg/ml.

10. Drinkable pharmaceutical composition in dosage units, **characterized in that** it comprises the aqueous solution according to Claim 1, in which the SR 57746 hydrochloride is present in an amount of from 0.5 to 10 mg per dosage unit.

11. Composition according to Claim 10, in which the SR 57746 hydrochloride is present in an amount of from 1 to 5 mg per dosage unit.

12. Composition according to Claim 11, **characterized in that** it comprises the aqueous solution according to Claim 2.

13. Composition according to Claim 11, **characterized in that** it comprises the aqueous solution according to Claim 3.

14. Composition according to Claim 11, **characterized in that** it comprises the aqueous solution according to Claim 4.

15. Composition according to Claim 11, **characterized in that** it comprises the aqueous solution according to Claim 5.

16. Composition according to Claim 11, **characterized in that** it comprises the aqueous solution according to Claim 6.

17. Composition according to Claim 11, **characterized in that** it comprises the aqueous solution according to Claim 7.

18. Drinkable pharmaceutical composition according to Claim 11, **characterized in that** it comprises 2.2 mg of SR 57746 hydrochloride, 40 mg of $\beta$-CD, 42 mg of citric acid monohydrate and water, the total volume of the composition being 4 ml.

19. Drinkable pharmaceutical composition according to Claim 11, **characterized in that** it comprises 4.4 mg of SR 57746 hydrochloride, 40 mg of $\beta$-CD, 42 mg of citric acid monohydrate and water, the total volume of the composition being 4 ml.

20. Complex formed between a molecule of SR 57746 and two molecules of $\beta$-CD.